# EUROPEAN PATENT APPLICATION

(11) **EP 4 603 595 A1**
(43) Date of publication of application: **20.08.2025**
(21) Application number: 24157316.1
(22) Date of filing: 13.02.2024
(51) Int. Cl.: C12Q 1/6853

(54) **A METHOD FOR TYPING THE IMMUNE GENES AND THE ALLELIC VARIANTS THEREOF**

(71) Applicant: ImmuneDiscover Sweden AB, 104 51 Stockholm (SE)
(72) Inventor: KARLSSON HEDESTAM, Gunilla, 115 20 STOCKHOLM (SE); CORCORAN, Martin, 112 55 STOCKHOLM (SE)
(74) Representative: Kransell & Wennborg KB

(57) **Abstract**

The present disclosure generally relates to a method (1) for typing the immune genes and the allelic variants thereof. The present disclosure also relates to a kit for use in the method and to a set of target primers for use in the method.

## Description

### TECHNICAL FIELD

The present disclosure generally relates to a method for typing the immune genes and the allelic variants thereof. The present disclosure also relates to a kit and to a set of target primers for use in the method.

### BACKGROUND

The immune system is a network of biological processes that protects an organism from diseases. The innate immune system is the body's first line of defense against germs entering the body. It responds with a set of receptors that recognize conserved structures of germs and foreign substances, which is why it is sometimes referred to as the "nonspecific" immune system. The adaptive immune system provides a tailored response to each stimulus by learning to recognize molecules it has previously encountered.

The immune genes, and particularly the genes of the adaptive immune system, play important roles in the recognition and subsequent immunological defense against pathogens and the development of immunological memory such that subsequent re-infection by the same pathogen results in a rapid immunological response that specifically targets the viral/bacterial pathogen.

This process forms the basis for the process of immunization, using attenuated pathogens or pathogen-derived antigens as a means of preventing subsequent infection from that specific pathogen.

The technique of immunization is one of the most effective public health measures and has helped control or eliminate many debilitating and frequently fatal diseases that are prevalent around the world. Despite this, for many diseases no effective vaccines have been produced, and for others, there are variations in efficacy of currently available vaccines, both on an individual and a population level. At least part of the reason underlying this is due to genetic differences in the genes most important in the response to pathogens, i.e. the immune genes, particularly the adaptive immune receptor genes comprising the Band T-cell receptor (BCR and TCR) genes and the major histocompatibility (MHC) genes.

Identification of differences in these genes between people is of particular importance since they may influence an individual's ability to respond to specific pathogens, their predisposition to autoimmune disorders and malignancies and their ability to generate a neutralizing antibody response following vaccination.

The various genomic loci encoding the immune genes, and particularly the genes of the adaptive immune system, are known to be difficult to directly sequence using standard short read genomic sequencing techniques. This is due to these loci containing numerous pseudogenes and other non-functional sequences that are highly similar to the functional adaptive immune genes. In addition, the loci have been subject to various duplication events during the course of human evolution, some of which have resulted in duplicated segments present in entirely different chromosomes. The presence of these non-functional duplications results in major difficulties in achieving accurate short read genomic sequence assembly in the adaptive immune loci. To date, no high throughput methodology exists that enables the identification of the full set of adaptive immune alleles present in an individual.

A hallmark of the adaptive immune system is the vast repertoire of B-cell receptors (BCR) and T-cell receptors (TCRs) a given individual expresses, each capable of recognizing a unique antigenic structure. The theoretical diversity of each of the B-cell and T cell repertoires is greater than the number of B-cells and T-cells in our bodies and has been estimated to correspond to somewhere from 10¹⁰ to 10¹⁴ specificities. To generate such enormous diversity, a combinatorial recombination process is required. The molecular basis for this recombination process was first revealed in the 80's and resulted in a Nobel Prize in Physiology or Medicine in 1987.

While the basic mechanisms of germline gene recombination to produce functional immune receptors were worked out some time ago, the methodology to study such big datasets at high resolution only became available recently with the advent of Next Generation Sequencing (NGS) techniques. However, even with the availability of NGS, several technological hurdles have remained to define the diversity of e.g. the BCR and TCR germline genes due to the unusual complexity of the genomic loci encoding these genes.

Both the BCR and TCR are generated from germline gene segments referred to as variable (V), diversity (D) and joining (J) segments. For the BCR, there are 107 V gene segments (52 for the heavy chain and 55 for the light chain split between the lambda and kappa light chains), 18 J segments (6 for the heavy chain and 12 for the light chains) and 27 D segments, i.e. 152 gene segments in total. For the TCR, there are 109 V genes (47 for the α chain, 48 for the β chain, 8 for the δ chain and 6 for the γ chain), 78 J segments and 6 D segments, i.e. 193 gene segments in total. This means that there are 345 gene segments that recombine to make up the BCR and TCR repertoires, and each segment exists in multiple allelic variants. Currently, there are approximately 250 alleles reported for the 52 heavy chain V genes and this is far from a complete list of alleles.

For the MHC molecules, there are considerably fewer genes encoding molecules responsible for antigen-presentation, only 9 (explaining why the MHC loci are better characterized), but there is a considerable number of allelic variants.

For each of the BCR, TCR and MHC genes, a person can be homozygote or heterozygote, which adds to the complexity. Furthermore, a given individual may encode deletions or duplications of one of more gene segments, adding further genetic diversity between individuals. Thus, the repertoires of germline immune genes and gene variants (alleles) differ significantly between individuals, a level of diversity that so far has not been possible to characterize due to the lack of efficient methods to sequence these regions in large numbers of subjects.

In view of this, there is a need to provide a method for typing the immune genes and the allelic variants thereof, in an efficient, rapid, and high-throughput manner.

### SUMMARY

In view of the above-mentioned problems, it is an object of the present disclosure to provide improvements in typing the immune genes and their allelic variants.

According to a first aspect, there is provided method for typing immune genes and allelic variants thereof comprising:
a) providing a set of amplified libraries in at least one first array comprising a plurality of wells by performing an individual amplification reaction in each well of the first array(s); the individual amplification reaction comprising contacting a genomic DNA sample with a polymerase and a set of target primers; the set of target primers comprising a plurality of reverse target primers; each reverse target primer targeting the 3' end of a pre-determined gene segment of an immune gene, and a plurality of forward target primers; each forward target primer targeting the 5' end of the pre-determined gene segment, wherein
   each of the reverse target primers comprises a reverse target sequence and a reverse universal sequence arranged 5' of the reverse target sequence, wherein the reverse universal sequence is common to all reverse target primers in the set of target primers, wherein
   each of the forward target primers comprises a forward target sequence and a forward universal sequence arranged 5' of the forward target sequence, wherein the forward universal sequence is common to all forward target primers in the set of target primers; whereby an individual amplified library is obtained in each well; the amplified library comprising a plurality of target regions comprising the targeted pre-determined gene segments, a reverse universal region corresponding to the reverse universal sequence, and a forward universal region corresponding to the forward universal sequence;
b) labelling each amplified library of the set of amplified libraries in a plurality of second arrays; each of the second arrays comprising a plurality of wells, by performing an individual amplification reaction in each well of the second arrays; the individual amplification reaction comprising contacting the amplified library with a polymerase, a well-index primer pair, and an array-index primer pair, wherein
   the well-index primer pair comprises a reverse well-index primer and a forward well-index primer; the reverse well-index primer comprising a first reverse well-universal sequence targeting the reverse universal region, a second reverse well-universal sequence, and a reverse well-specific index sequence arranged between the first and second reverse well-universal sequences; the forward well-index primer comprising a first forward well-universal sequence targeting the forward universal region, a second forward well-universal sequence, and a forward well-specific index sequence arranged between the first and second forward well-universal sequences, wherein
   the array-index primer pair comprises a reverse array-index primer and a forward array-index primer; the reverse array-index primer comprising at least a first reverse array-universal sequence targeting the second reverse well-universal sequence; the forward array-index primer comprising at least a first forward array-universal sequence targeting the second forward well-universal sequence, wherein at least one of the reverse and forward array-index primers comprises an array-specific index sequence arranged 5' of the first reverse array-universal sequence or the first forward array-universal sequence; wherein
   different well-index primer pairs are used in each well of the second arrays, and wherein identical array-index primer pairs are used in each well of each individual second array of the plurality of second arrays,
      whereby a labelled amplified library is obtained in each well,
c) combining the labelled amplified libraries of each well of the plurality of second arrays to yield a combined library;
d) sequencing the combined library to yield a sequenced library;
e) analyzing the sequenced library.

The method of the present disclosure allows for a significantly improved understanding of the genetic variations of the immune genes. The typing; i.e. individual characterization of the various alleles of the immune genes may provide invaluable information about the role of the immune system, e.g. the adaptive immune system, in health and disease, and opens up entirely new opportunities in human immunology.

The method of the present disclosure offers a solution to create full profiles of the immune genes such that a personalized genotyping of e.g. the adaptive immune genes can be accomplished in a robust and rapid manner.

The method is a high throughput method and allows for a large and cohort evaluation to be provided. By means of the method of the present disclosure, associations between genetic polymorphisms in immune genes and development or protection against diseases may be revealed. For example, the presence of certain antibody V alleles may predispose to improved antibody responses, such as shown for the VH1-2*02 allele required to make a certain class of broadly neutralizing anti-HIV antibodies or the VH1-69*20 allele used in neutralizing antibody responses against SARS-CoV-2.

Knowledge about which alleles a given person has will open possibilities for personalized vaccine strategies aimed to induce specific classes of antibodies. Other alleles may predispose to the development of auto-antibody responses (such as those seen in e.g. rheumatoid arthritis, MS or lupus). In these cases, knowledge of which germline alleles an individual has will have diagnostic value and potentially also therapeutic value as such receptors could be selectively targeted for elimination.

The method of the present disclosure involves the application of a targeted genomic library amplification approach to map and identify the allelic variants of the genetic components of the immune system, preferably the adaptive immune system. In this way a plurality of target gene segments can be simultaneously amplified per sample.

In the first step a) of the method, amplification is performed in separate wells of at least one first array.

An individual genomic DNA sample is utilized in each well of the first array. The individual genomic DNA sample constitutes the total genetic information of an individual, and thus contains the genes of interest, i.e. the immune genes, preferably the genes of the adaptive immune system.

Different genomic DNA samples are typically utilized in the separate wells of the first array.

The inventors have developed a set of target primers, which are designed to promote amplification of the immune genes. The set of target primers are designed to amplify predetermined gene segments of the immune genes.

The set of target primers comprises a plurality of forward primers, and reverse target primers. The forward and reverse target primers bind to opposite strands of the template DNA, at the edges of the region to be copied. A polymerase, e.g. DNA polymerase is used to synthesize new strands of DNA complementary to the template strand.

Each individual genomic DNA sample is contacted by a polymerase, typically a DNA polymerase, and a set of target primers under conditions that allow for amplification to be accomplished in each well of the first array.

Amplification is typically accomplished by first denaturing the doublestranded DNA templates, e.g. by heating, annealing the templates, wherein the primers bind to the flanking region of the target DNA, and extension, in which the DNA polymerase extends the primer along the template strands. These steps may be repeated multiple times to produce exact copies of the target DNA.

This way, an amplified library containing the target regions of interest is obtained in each well of the first array. The amplified library comprises a plurality of target regions complementary to the targeted pre-determined gene segments, a reverse universal region corresponding to the reverse universal sequence, and a forward universal region corresponding to the forward universal sequence.

The first step of the method of the present disclosure allows for multiple amplifications involving a plurality of genomic DNA samples to be carried out simultaneously in one single run.

The first amplification step is typically carried out by polymerase chain reaction (PCR).

Each target primer contains a target sequence configured to hybridize to the predetermined gene segment or to a region upstream or downstream of the predetermined gene segment. Hence, the various target primers contain different target sequences designed to amplify different gene segments of the immune genes.

Furthermore, each target primer in the set of target primers contains a respective universal sequence. The universal sequences are common to all target primers in the set of target primers and are used in the subsequent step of labelling the amplified libraries with well-specific indexes. Accordingly, the universal sequences introduced in the amplified library provide a means to apply an indexing "bridge" during further amplifications, and thereby extend and label the amplified libraries obtained in step a).

The reverse target primer comprises a reverse universal sequence. The reverse universal sequence is arranged 5' of the reverse target sequence. Accordingly, the reverse universal sequence forms the 5' end (or "outer" end) of the reverse target primer. The reverse universal sequence allows for the subsequent labelling with a reverse well-specific index sequence.

The forward target primer comprises a forward universal sequence. The forward universal sequence is arranged 5' of the forward target sequence. Accordingly, the forward universal sequence forms the 5' end (or "outer" end) of the forward target primer. The forward universal sequence allows for the subsequent labelling with a forward well-specific index sequence.

The method of the present disclosure may comprise a step of purifying the amplified libraries obtained in step a) prior to step b) of labelling the amplified libraries.

This is to remove potential unincorporated primers.

The purification may be performed by means known to the skilled person.

The method of the present disclosure may further comprise a step of transferring the amplified libraries obtained in each well of the first array to separate wells of a plurality of second arrays, each comprising a plurality of wells.

The first array may be a first PCR plate. The plurality of second arrays may be a plurality of second PCR plates.

In step b) each amplified library contained in the wells of the second array is labelled with a well-specific index sequence and an array-specific index sequence.

In the labelling step, individual amplification reactions are carried out in each well of the plurality of second arrays.

Similar to step a), multiple amplifications can be carried out simultaneously in one single run, typically by means of PCR amplification.

The well-index primer pair comprises a reverse well-index primer and a forward well-index primer.

Each of these well-index primers contains a well-specific index sequence, and two universal sequences; i.e. two well-universal sequences.

The reverse well-index primer comprises a reverse well-specific index sequence, a first reverse well-universal sequence and a second reverse well-universal sequence. The reverse well-specific index sequence is arranged between the first reverse well-universal sequence and the second reverse well-universal sequence.

The second reverse well-universal sequence forms the 5' end (or "outer" end) of the reverse well-index primer. The second reverse well-universal sequence is subsequently targeted by the reverse array-index primer, which allows an array-specific index sequence to be introduced. Hence, the second reverse well-universal sequence provides a means to apply an additional indexing "bridge" during further amplifications.

The first reverse well-universal sequence targets the reverse universal region of the amplified library obtained in step a). In other words, the first reverse well-universal sequence is configured to hybridize to the reverse universal region of the amplified library obtained in step a).

The same second reverse well-universal sequence is used for each reverse well-index primer.

The forward well-index primer comprises a forward well-specific index sequence, a first forward well-universal sequence and a second forward well-universal sequence. The forward well-specific index sequence is arranged between the first forward well-universal sequence and the second forward well-universal sequence.

The second forward well-universal sequence forms the 5' end (or "outer" end) of the forward well-index primer. The second forward well-universal sequence is subsequently targeted by the forward array-index primer, which allows an array-specific index sequence to be introduced. Hence, the second forward well-universal sequence provides a means to apply an additional indexing "bridge" during further amplifications.

The first forward well-universal sequence targets the forward reverse universal region of the amplified library obtained in step a). In other words, the first forward well-universal sequence is configured to hybridize to the forward universal region of the amplified library obtained in step a).

The same second forward well-universal sequence is used for each forward well-index primer.

The reverse well-specific index is specific for a particular well of the second arrays. Likewise, the forward well-specific index is specific for a particular well of the second arrays.

The well-index primer pairs secure that all samples stemming from a specific well can be identified in a subsequent analysis step, which is typically a bioinformatics analysis step.

This allows for the extraction of an amplified library belonging to a specific well.

The amplified libraries are furthermore labelled with an array-specific index by means of an array-index primer pair.

The array-index primer pair comprises a reverse array-index primer and a forward array-index primer.

The reverse array-index primer comprises at least a first reverse array-universal sequence. The reverse array-index primer targets the reverse well-universal sequence.

The forward array-index primer comprises at least a first forward array-universal sequence. The forward array-index primer targets the forward well-universal sequence.

At least one of the forward and reverse array-index primers comprises an array-specific index sequence. The array-specific index sequence is arranged 5' of the first reverse or first forward array-universal sequence.

Accordingly, the array-specific index sequence may be a forward array-specific index sequence or a reverse array-specific index sequence.

The array-specific index sequence secures that all samples stemming from a specific second array can be identified.

This allows for the extraction of all amplified libraries belonging to a specific second array in subsequent analysis steps of the sequenced library obtained in step d).

As mentioned hereinbefore, the well-index primer pair is different for each well of the second arrays. The array-index primer pair is common for each well of an individual second array, but different between the second arrays of the plurality of second arrays.

The labelling step b) allows for a labelled amplified library to be obtained in each well of the second arrays.

The universal regions of the amplified libraries in step a) allow the template to be extended by the well-index primers, and to secure that a reverse and a forward well-index sequence can be added. The extended sequence, in turn forms a new template for the "outer primers"; i.e. the array-specific index primers. The labelling step may thus be two sequential PCR reactions that occur in the same procedure, with the result that the original template acquires a forward well-specific index sequence, a reverse well-specific index, as well as an array-specific index.

Each labelled amplified library obtained in step b) thus comprises a plurality of target regions, two well-specific-indexes, which flank the target regions, as well as at least one array-specific index.

The method of the present disclosure may comprise a step of purifying the labelled amplified libraries obtained in step b) prior to step c) of combining the labelled amplified libraries.

The purification may be performed by means known to the skilled person.

In step c) of the method of the present disclosure, the labelled amplified libraries of each well of the second arrays are combined. Hence, a combined library comprising a plurality of sub-libraries (i.e. the individual amplified libraries) is obtained.

The combined library is a multiplexed product comprising multiple sets of labelled amplified libraries, each containing a set of target regions. Each set of target regions is associated with the amplification performed in one individual well of the first array. Since each amplified library is labelled with two well-specific index sequences, and with an array-specific sequence, an individual sub-library (i.e. an individual amplified library) contained in the combined library may be analyzed and compared with different sub-libraries and/or with a predefined gene reference set.

In step d) of the method of the present disclosure, the combined library is sequenced. Accordingly, a sequenced library is obtained.

In step e), the sequenced library is analyzed, preferably by bioinformatic analysis.

In exemplary embodiments, the reverse array-index primer may further comprise a second reverse universal sequence arranged 5' of the first reverse array-universal sequence; the forward array-index primer further comprising a second forward array-universal sequence arranged 5' of the first forward array-universal sequence, wherein the array-specific index sequence is arranged between the first and second reverse array-universal sequences or between the first and second forward array-universal sequences.

Accordingly, the second reverse array-universal sequence forms the 5' end (or "outer end") of the reverse array-index primer.

The second forward array-universal sequence forms the 5' end (or "outer end") of the forward array-index primer.

These "outer" universal sequences facilitate the introduction of the array-specific index sequence.

The sequencing step d) allows for the determination of the nucleic acids of each target sequence encoding a predetermined gene segment.

The sequencing step d) may comprise providing a reverse sequencing primer targeting the reverse array-index primer and a forward sequencing primer targeting the forward array-index primer.

The reverse sequencing primer may target the first reverse array-universal sequence or the second reverse array-universal sequence.

Likewise, the forward sequencing primer may target the first forward array-universal sequence or the second forward reverse array-universal sequence.

The sequencing may be carried out by next-generation sequencing (NGS). Next-generation sequencing (NGS) is a massively parallel sequencing technology with high throughput, scalability, and speed. The sequencing may be carried out in a sequencing device in one single sequencing run. Hence, the multiple amplifications performed in the various steps of the method may be sequenced in one single run.

The method of the present disclosure enables the typing; i.e. individual characterization of the genes of the immune system, and the allelic variants of these genes with a rapid and high-throughput approach.

Each labelled amplified library may comprise all the target regions associated with one DNA sample (and one well). Each labelled amplified library will be flanked or tagged by a well-specific index sequence on each side of the target region. Furthermore, each labelled amplified library will be tagged with an array-specific index sequence.

As mentioned hereinbefore, the well-specific indexes allow for the extraction of the target sequences associated with a specific amplified library (associated with a specific well and a specific genomic DNA sample). The array-specific index allows for the extraction of each amplified library of one second array.

For example, the multiplexed products in well 1; i.e. the targeting of multiple adaptive immune loci genes from one initial template/individual, will contain one forward well-specific index sequence and a separate reverse well-specific index sequence that labels that library as being located in well position 1.

The library in well 2 will likewise be labelled with a forward "well 2"-specific index sequence and a reverse "well 2"-specific index seqence and so on. All the libraries of the first set of amplified libraries will thus have two well-specific index sequences present that can enable the subsequent identification of the specific well position of each sequence of that library.

For example, if one second array comprises 100 wells, then one second array will contain 100 sub-libraries. The identification of the forward well-specific index sequences and the corresponding reverse well-specific index sequences enables the identification of each of the 100 sub-libraries. Since each of these sub-libraries are derived from the targeted amplification of a set of the adaptive immune genes of a single individual, it is possible to utilize these targeted libraries to derive the adaptive immune genotype of each of the 100 individuals.

Hence, the target regions belonging to a specific well may be extracted and evaluated and compared with corresponding target regions of different wells within the plurality of second arrays. Furthermore, the array-specific index allows for a plurality of arrays to be utilized simultaneously and for subsequent analysis of individual arrays to be carried out.

The step b) of labelling the amplified libraries of the first set of amplified libraries is performed in a plurality of different second arrays comprising a plurality of wells.

For example, the plurality of second arrays may comprise between 3 and 400, preferably between 10 and 250 different second arrays.

The first array may e.g. be a PCR plate comprising 96 different wells. Likewise, the second array may be a PCR plate comprising 96 different wells. If e.g. 20 different second arrays are utilized, 1920 individual cases (96x24) may be simultaneously sequenced and analyzed, for example on an Illumina MiSeq instrument or an Illumina HiSeq instrument. Accordingly, the method of the present disclosure offers a high-throughput analysis of the immune genes and its allelic variants.

The total number of second arrays included in each sequencing run is typically dependent on the overall sequence capacity of the sequencing device utilized.

Preferably, the immune genes are genes of the adaptive immune system.

As mentioned hereinbefore, these genes are known for their massive variety of allelic variants. These genes are also the genes of the immune system that are considered to be the most important in the defense against invading pathogens and toxic substances produced by such pathogens. Hence, the knowledge about which alleles an individual person expresses offers opportunities for personalized vaccine strategies aimed at inducing specific antibodies.

The immune genes may be B-cell receptor (BCR) genes, T-cell receptor (TCR) genes or major histocompatibility (MHC) genes. Preferably, the immune genes are B-cell receptor (BCR) genes, T-cell receptor (TCR) genes.

B-cells and T-cells play key roles in the adaptive immune response. B-cells act as antigen-presenting cells and produce antibodies. The antibodies bind to foreign substances produced by pathogens, such as toxins, to neutralize them. Antibodies also bind directly to viruses and block their ability to enter new host cells, thereby controlling infections.

The role of the T-cells is to directly kill infected host cells, activate other immune cells, produce cytokines, and regulate the immune response.

The B-cell receptor (BCR) is a transmembrane protein on the surface of a B-cell, which controls the activation of the B-cell.

The T-cell receptor (TCR) is a transmembrane protein on the surface of T-cells, which controls the activation of the T-cell. The TCR binds to antigen fragments (peptides) presented by MHC molecules on e.g. abnormal cells, cancer cells, and infected cells. This interaction causes the T cells to attack these cells and helps the body fight infection.

The BCR genes include the IGH, IGK and IGL genes. The TCR genes include the TCRA, TCRB, TCRD and TCRG genes.

The MHC genes consists of the MHC class I and class II genes, which encode molecules that present peptides to CD8+ and CD4+ T-cells, respectively.

A mentioned hereinbefore, a plurality of different variable (V), diversity (D), junction (J) or constant (C) gene segments make up the BCR and TCR receptors.

Accordingly, the predetermined gene segment may comprise a gene segment of the variable (V), diversity (D), junction (J) or constant (C) B-cell or T-cell receptor genes, or of MHC gene exons.

As mentioned hereinbefore, the vast number of genes (e.g. V, D, J and constant genes) making up the BCR and TCR exist in multiple allelic variants.

In this regard, the method of the present disclosure allows for a plurality of target gene segments to be simultaneously amplified per sample, enabling e.g. an immune receptor genotype to be identified from the amplified library of sequences for each case.

In exemplary embodiments, the predetermined gene segment of the immune gene is between 100 and 1000 bp in size, preferably between 300 and 800 bp in size.

This range renders the technique applicable to use with sequencing instruments like the Illumina HiSeq or Illumina MiSeq.

However, the method of the present disclosure is not limited to this size range. Instruments having higher capacity exist on the market and are subject to further development. A larger size of the predetermined gene segment is thus conceivable.

In exemplary embodiments, the ratio of the well-index primer pairs to the array-index primer pairs is at least 1: 10, preferably from 1:20 to 1:60.

The well-index primers are rate-limiting, and are typically used in lower concentrations. The array-index primers bind to the inner primer reaction. The array-index primers are typically used in higher concentrations.

In exemplary embodiments, the set of target primers may comprise from 5 to 250, preferably from 15 to 150 reverse and forward target primers.

The number of target primers in the set of target primers depends on the analysis to be performed. In some scenarios, a smaller number of target primers may be sufficient, whereas a larger number is preferably used to enable a more full-scale analysis.

If a more comprehensive analysis is to be performed, a range of from 15 to 150, preferably from 30 to 150, reverse and forward target primers capture the most important gene segments and genes of the immune system, preferably the genes of the adaptive immune system.

It is worth noting that an individual reverse or forward target primer can target more than one gene segment or gene. Accordingly, the number of individual forward and reverse primers required to target a set of target genes can be less than the number of targets.

In exemplary embodiments, at least one of the reverse or forward target primers of the set of target primers may further comprise a unique molecular identifier (UMI) sequence.

A unique molecular identifier (UMI) is a type of molecular barcode that provides error correction and increased accuracy during sequencing. The molecular barcode is a short sequence used to uniquely tag each molecule in a sample library. The introduction of a UMI sequence may alleviate PCR duplication errors.

Furthermore, in the method of the present disclosure, the UMI sequence allows for the use of lower concentrations of template DNA.

In exemplary embodiments, step e) comprises a bioinformatic analysis of the data obtained in step d), wherein the bioinformatic analysis comprises identifying the allelic variants of the genes of the immune system by comparing the target regions of each labelled amplified library with a predefined gene reference set.

In this post-sequencing step, the target regions associated with a specific gene segment or gene may be compared between the various individuals, i.e. between the various amplified libraries. This is accomplished by first extracting the amplified libraries belonging to a specific second array by using the array-specific index, and subsequently extracting the amplified libraries belonging to a specific well by using the well-specific indexes.

The nucleotide sequences of a specific targeted sequence (target region) in one amplified library may be compared with the same targeted sequence (target region) in the other amplified libraries to identify similarities and differences. This allows for the determination of the allelic variants of a specific gene segment and/or a specific gene.

The target regions may be compared with a pre-defined gene reference set.

The pre-defined gene reference set may form part of a bioinformatic database of the currently known variants of the target genes.

A bioinformatic analysis of the sequenced libraries obtained by the method, as defined hereinabove, may enable the identification of both known and novel variants.

In exemplary embodiments, step e) is performed by a processing unit.

The processing unit may in some embodiments form part of a computer system, further comprising a database. The computer system may be implemented as a server. It may be possible to make use of more than a single processing unit, such as for example including FPGAs, ASICs, etc., software systems and applications, software packages, mechanical and electrical parts, etc. Software packages may be part of the server, the database, and/or a network.

The extracted sub-libraries may be assigned to a database of allelic variants for each of the targeted adaptive immune genes. Accordingly, a rapid processing of the sub-library sequences is accomplished.

The processing unit may be configured to identify both novel and known variants.

Hence, the processing unit can use a pre-defined reference database and type the presence of known alleles in each sample analyzed. It can also identify new, previously unknown alleles for the genes examined, and in that case, the final genotype for each case will include both known and novel variants.

According to another aspect, there is provided kit for use in a method of typing the immune genes and the allelic variants thereof, wherein the kit comprises:
- a set of target primers comprising a plurality of reverse target primers; each reverse target primer targeting the 3' end of a pre-determined gene segment of an immune gene, and a plurality of forward target primers; each forward target primer targeting the 5' end of the pre-determined gene segment, wherein each of the reverse target primers comprises a reverse target sequence and a reverse universal sequence arranged 5' of the reverse target sequence, wherein the reverse universal sequence is common to all reverse target primers in the set of target primers, wherein each of the forward target primers comprises a forward target sequence and a forward universal sequence arranged 5' of the forward target sequence, wherein the forward universal sequence is common to all forward target primers in the set of target primers;
- a plurality of well-index primer pairs, wherein each well-index primer pair comprises a reverse well-index primer and a forward well-index primer; the reverse well-index primer comprising a first reverse well-universal sequence corresponding to the reverse universal sequence of the reverse target primer, a second reverse well-universal sequence, and a reverse well-specific index sequence arranged between the first and second reverse well-universal sequences; the forward well-index primer comprising a first forward well-universal sequence corresponding to the forward reverse universal sequence of the forward target primer, a second forward well-universal sequence, and a forward well-specific index sequence arranged between the first and second forward well-universal sequences,
- a plurality of array-index primer pairs each comprising a reverse array-index primer and a forward array-index primer; the reverse array-index primer comprising at least a first reverse array-universal sequence targeting the second reverse well-universal sequence; the forward array-index primer comprising at least a first forward array-universal sequence targeting the second forward well-universal sequence, wherein at least one of the reverse and forward array-index primers comprises an array-specific index sequence arranged 5' of the a first reverse array-universal sequence or the first forward array-universal sequence.

The kit may be used in the method described hereinbefore.

The kit (and the method) of the present disclosure may be used to analyze variations in specific immune genes. For example, variations in adaptive immune receptor genes that predisposes to autoimmune diseases, such as for example multiple sclerosis (MS), rheumatoid arthritis (RA), inflammatory bowel disease (IBD) or systemic lupus erythematosus (SLE), may be identified.

Furthermore, the kit (and the method) may be used to identify individuals able to respond to a specific vaccine or vaccine strategy, such as a vaccine against Haemophilus influenzae type b (Hib) or HIV.

According to another aspect, there is provided a set of target primers comprising a plurality of reverse target primers and a plurality forward target primers for use in a method of typing the immune genes, preferably the genes of the adaptive immune system, and the allelic variants thereof, wherein
the set of target primers may be configured to target the IGHD genes and comprises the reverse target primers defined by SEQ ID NO: 1-13 or corresponding sequences having at least 95 %, preferably at least 99% sequence homology with SEQ ID NO: 1-13, and the forward target primers defined by SEQ ID NO: 14-31 or corresponding sequences having at least 95%, preferably at least 99 % sequence homology with SEQ ID NO: 14-31, and/or wherein
the set of target primers may be configured to target the IGHJ genes and comprises the reverse target primers defined by SEQ ID NO: 32-36 or corresponding sequences having at least 95%, preferably at least 99 % sequence homology with SEQ ID NO:32-36, and the forward target primers defined by SEQ ID NO: 37-41 or corresponding sequences having at least 95%, preferably at least 99 % sequence homology with SEQ ID NO:37-41, and/or wherein
the set of target primers may be configured to target the IGHV genes and comprises the reverse target primers defined by SEQ ID NO:42-74 or corresponding sequences having at least 95%, preferably at least 99 % sequence homology with SEQ ID NO:42-74, and the forward target primers defined by SEQ ID NO: 75-121 or corresponding sequences having at least 95%, preferably at least 99 % sequence homology with SEQ ID NO:75-121, and/or wherein
the set of target primers may be configured to target the TRG genes and comprises the reverse target primers defined by SEQ ID NO: 122-139 or corresponding sequences having at least 95%, preferably at least 99 % sequence homology with SEQ ID NO: 122-139, and the forward target primers defined by SEQ ID NO: 140-157 or corresponding sequences having at least 95%, preferably at least 99 % sequence homology with SEQ ID NO: 140-157.

The set of target primers may be used in the method described hereinbefore.

The set of target primers may be used to analyze a specific gene category (e.g. the IGHJ, IGHD genes etc.) and make an individual comparison between a plurality of individuals.

Alternatively, the set of target primers may be used to generate a more comprehensive profile and individually comparing such a profile between a plurality of individuals. In this case, all the reverse and target primers defined in SEQ ID NO: 1-157 may be utilized.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1a conceptually illustrates the steps associated with an exemplary embodiment of the method of the present disclosure.
Figure 1b conceptually illustrates the steps associated with an alternative embodiment of the method of the present disclosure, wherein a UMI sequence is introduced.
Figure 2 illustrates the immunoglobulin heavy chain variable (IGHV) genotype output of eight individuals using the method of the present disclosure. Eight cases were genotyped using the method of the present disclosure and the allelic variants of their IGHV genes are shown, illustrating the great allelic variation in these genes between individuals. The presence of a particular IGHV gene variant is represented by a black box and the absence is represented by a white box.

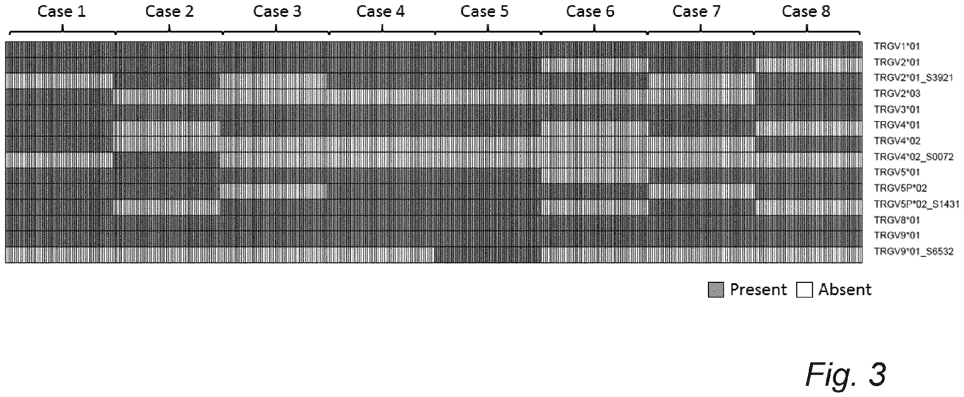

Figure 3 illustrates the TRGV genotype replicates in eight individuals. Eight cases were each independently genotyped 50 times using the method of the present disclosure. The T cell receptor gamma variable (TRGV) genotype of all 50 replicates of the 8 cases are shown, illustrating the consistency of the output of genotypes derived with the method of the present disclosure. Each of the eight cases shows a distinct TRGV allelic composition and all 50 replicates for each case show the same set of allelic variants. The presence of a TRGV allele gene variant is represented by a gray box and the absence by a white box.
Figures 4a and 4b illustrate adaptive immune genes that may be targeted by the target primers of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

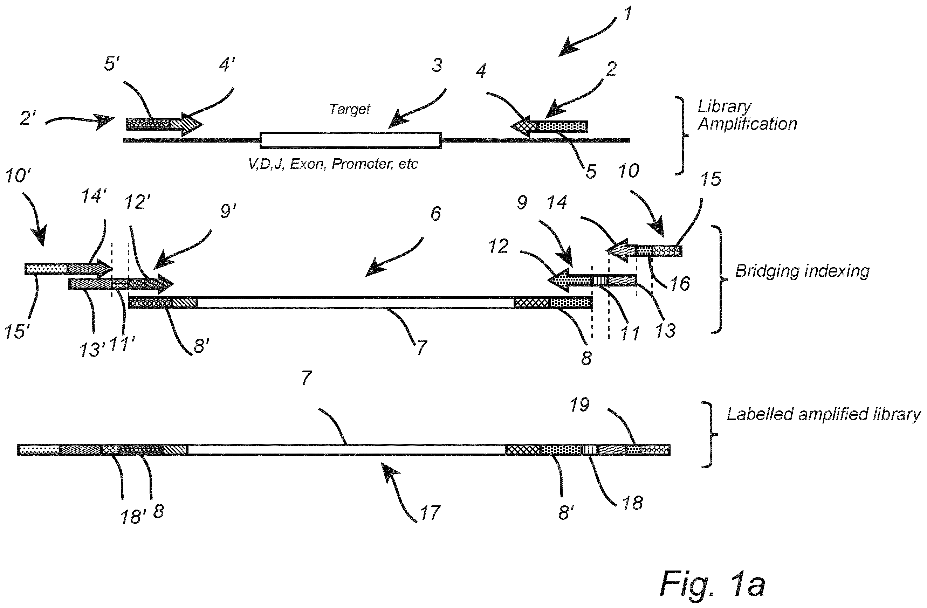

Figure 1a conceptually illustrates the method for typing the immune genes and the allelic variants thereof according to the present disclosure. The method 1 comprises:
a) providing a set of amplified libraries 6 in at least one first array comprising a plurality of wells by performing an individual amplification reaction in each well of the first array(s); the individual amplification reaction comprising contacting a genomic DNA sample with a polymerase and a set of target primers; the set of target primers comprising a plurality of reverse target primers 2; each reverse target primer targeting the 3' end of a pre-determined gene segment 3 of an immune gene, and a plurality of forward target primers 2'; each forward target primer targeting the 5' end of the pre-determined gene segment 3, wherein
   each of the reverse target primers 2 comprises a reverse target sequence 4 and a reverse universal sequence 5 arranged 5' of the reverse target sequence 4, wherein the reverse universal sequence 5 is common to all reverse target primers 2 in the set of target primers, wherein
   each of the forward target primers 2' comprises a forward target sequence 4' and a forward universal sequence 5' arranged 5' of the forward target sequence 4', wherein the forward universal sequence 5' is common to all forward target primers 2 in the set of target primers;
   whereby an individual amplified library 6 is obtained in each well; the amplified library 6 comprising a plurality of target regions 7 comprising the targeted pre-determined gene segments 3, a reverse universal region 8 corresponding to the reverse universal sequence 5, and a forward universal region 8' corresponding to the forward universal sequence 5';
b) labelling each amplified library 6 of the set of amplified libraries 6 in a plurality of second arrays; each of the second arrays comprising a plurality of wells, by performing an individual amplification reaction in each well of the second arrays; the individual amplification reaction comprising contacting the amplified library 6 with a polymerase, a well-index primer pair (9,9'), and an array-index primer pair (10,10'), wherein
   the well-index primer pair (9,9') comprises a reverse well-index primer 9 and a forward well-index primer 9'; the reverse well-index primer 9 comprising a first reverse well-universal sequence 12 targeting the reverse universal region 8, a second reverse well-universal sequence 13, and a reverse well-specific index sequence 11 arranged between the first 12 and second 13 reverse well-universal sequences; the forward well-index primer 9' comprising a first forward well-universal sequence 12' targeting the forward universal region 8', a second forward well-universal sequence 13', and a forward well-specific index sequence 11' arranged between the first 12' and second 13' forward well-universal sequences, wherein
   the array-index primer pair (10,10') comprises a reverse array-index primer 10 and a forward array-index primer 10'; the reverse array-index primer 10 comprising at least a first reverse array-universal sequence 14 targeting the second reverse well-universal sequence 13; the forward array-index primer 10' comprising at least a first forward array-universal sequence 14' targeting the second forward well-universal sequence 13', wherein at least one of the reverse 10 and forward 10' array-index primers comprises an array-specific index sequence 16 arranged 5' of the first reverse array-universal sequence 14 or the first forward array-universal sequence 14'; wherein
   different well-index primer pairs (9,9') are used in each well of the second arrays, and wherein identical array-index primer pairs (10, 10') are used in each well of each individual second array of the plurality of second arrays,
   whereby a labelled amplified library 17 is obtained in each well,
c) combining the labelled amplified libraries 17 of each well of the plurality of second arrays to yield a combined library;
d) sequencing the combined library to yield a sequenced library;
e) analyzing the sequenced library.

As used herein, the term "immune gene" means any gene of the immune system. Preferably, the immune gene is a gene related to the adaptive immune system. Hence, the immune genes may be genes of the adaptive immune system or genes interacting with the adaptive immune genes.

For example, the immune genes may be genes of the B-cell receptor (BCR), the T-cell receptor (TCR) or major histocompatibility (MHC) genes.

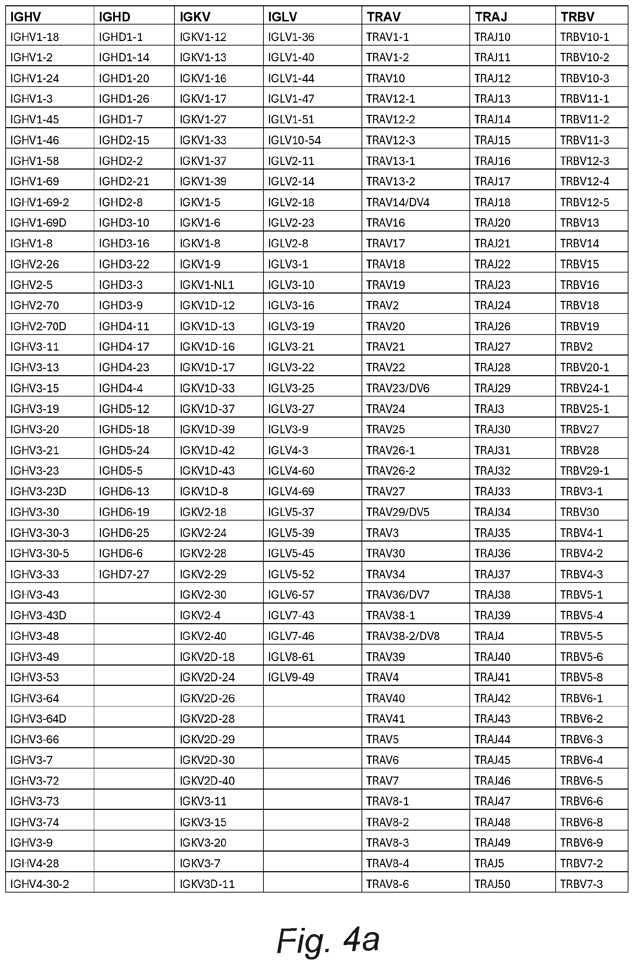

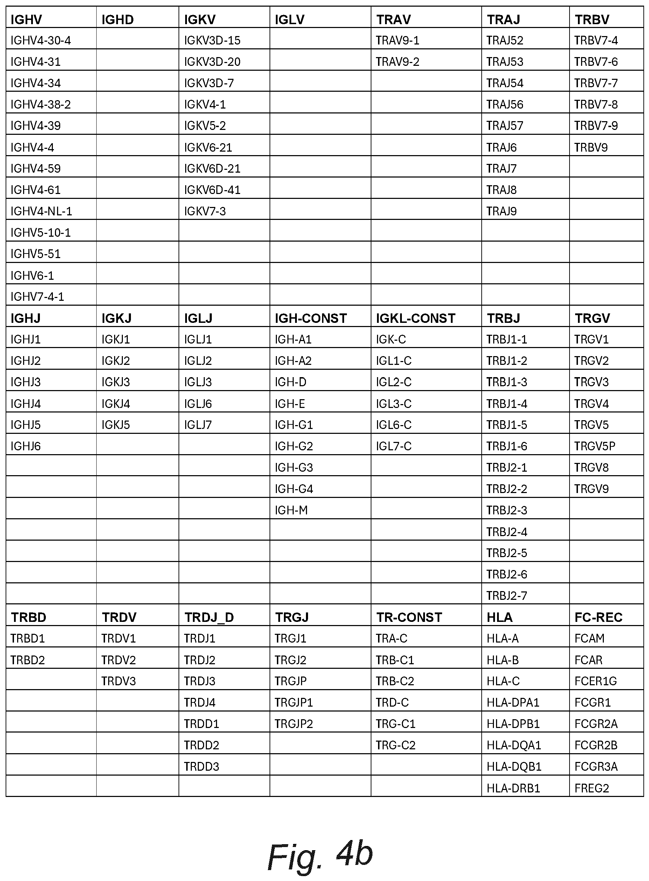

Figures 4a and 4b list adaptive immune receptor genes which may be targeted by the target primers of the method of the present disclosure. The adaptive immune receptor genes include the variable (V), diversity (D), and junction (J) genes of the B cell receptor (BCR/immunoglobulin (IG)) from the IG heavy chain (IGH), kappa chain (IGK) and lambda chain (IGL), and the V, D and J genes of the T cell receptor TCR from the alpha chain (TRA), beta chain (TRB), delta chain (TRD) and the gamma chain (TRG). The target primers of the method of the present disclosure also target the corresponding constant exons of these genes and sets of genes that include the HLA genes and the FC receptor genes, which closely interact with the adaptive immune receptor genes.

"Typing" the immune genes and the allelic variants thereof means determining differences in the genotype of an individual by examining the individual's DNA sequences and comparing it to other individuals' sequences or reference sequences. Hence, the typing allows for an individual characterization of the immune genes and the allelic variants thereof.

The "predetermined gene segment" is a gene segment of an immune gene targeted by the target-specific primers. The predetermined gene segment may comprise a gene segment of the variable (V), diversity (D), the junction (J), or constant (C) genes of the B-cell or T-cell receptor genes, or MHC gene exons.

The term "genomic DNA sample" means a sample that contains the genes of interest, i.e. the immune genes, preferably the genes of the adaptive immune system. The genomic DNA sample constitutes the genetic information of an individual. The genomic DNA sample may also be referred to as a genomic DNA template. The genomic DNA sample may e.g. be extracted from blood, saliva, swabs, tissue biopsies, dried blood spots or cultured cells.

In the first step a) of the method, an individual genomic DNA sample is contacted with a polymerase, typically a DNA polymerase and a set of target primers.

Individual amplification reactions are performed in separate wells of a first array.

The first array may e.g. be a PCR plate.

As illustrated in figure 1a, the set of target primers contains at least one reverse target primer 2 and a forward target primer 2'. Each of the target primers contains a target-specific sequence 4,4', and a universal sequence 5,5'.

The "universal sequence" is a sequence which enables a link or "bridge" to subsequent amplifications in step b) of labelling the amplified library 6 obtained in step a).

The reverse universal sequence 5 is common to all reverse target primers 2 used in the method. Likewise, the forward universal sequence 5' is common to all forward target primers 2' used in the method.

The reverse universal sequence 5 is typically different from the forward universal sequence 5'. This allows the subsequent labelling or "bridging" step to be specific to one or the other end.

The reverse universal sequence 5 of the reverse target primer 2 enables the introduction of a reverse universal region 8 in each amplified library of the wells of the first array (step a). The reverse universal region 8 forms the "bridge" or link to subsequent amplifications. The reverse well index primer 9 is configured to hybridize to the reverse universal region 8.

The reverse universal sequence 5 forms the 5' end of the reverse target primer 2. The 5' end of the reverse target primer may also be referred to as the "outer end" of the reverse target primer.

Likewise, the forward universal sequence 5' of the forward target primer 2' enables the introduction of a forward universal region 8' in each amplified library of the wells of the first array (step a). The forward universal region 8' forms the "bridge" or link to subsequent amplification. The forward well index primer 9' is configured to hybridize to the forward universal region 8'.

The forward universal sequence 5' forms the 5' end of the forward target primer 2'. The 5' end of the forward target primer may also be referred to as the "outer end" of the forward target primer 2'.

The method involves a "set of target primers", which means a plurality of reverse target primers and a plurality of forward target primers. Each of the reverse target primers contains a reverse target sequence 4 which is specific to that reverse target primer. Likewise, each of the forward target primers contains a forward target sequence 4', which is specific to that forward target primer.

The set of target primers may comprise a plurality of reverse target primers and forward target primers targeting a specific gene, a specific category of genes or a plurality of genes and/or gene categories of the adaptive immune system.

The set of target primers may be used to analyze a plurality of genes of the adaptive immune system.

The set of target primers may be configured to target the IGHD genes and may comprise the reverse target primers defined by SEQ ID NO:1-13 or corresponding sequences having at least 95 %, preferably at least 99% sequence homology with SEQ ID NO:1-13, and the forward target primers defined by SEQ ID NO:14-31 or corresponding sequences having at least 95%, preferably at least 99 % sequence homology with SEQ ID NO:14-3.

Alternatively, or in addition, the set of target primers may be configured to target the IGHJ genes and may comprise the reverse target primers defined by SEQ ID NO: 32-36 or corresponding sequences having at least 95%, preferably at least 99 % sequence homology with SEQ ID NO:32-36, and the forward target primers defined by SEQ ID NO: 37-41 or corresponding sequences having at least 95%, preferably at least 99 % sequence homology with SEQ ID NO:37-41.

Alternatively, or in addition, the set of target primers may be configured to target the IGHV genes and may comprise the reverse target primers defined by SEQ ID NO:42-74 or corresponding sequences having at least 95%, preferably at least 99 % sequence homology with SEQ ID NO:42-74, and the forward target primers defined by SEQ ID NO: 75-121 or corresponding sequences having at least 95%, preferably at least 99 % sequence homology with SEQ ID NO:75-121.

Alternatively, or in addition, the set of target primers may be configured to target the TRG genes and may comprise the reverse target primers defined by SEQ ID NO: 122-139 or corresponding sequences having at least 95%, preferably at least 99 % sequence homology with SEQ ID NO: 122-139, and the forward target primers defined by SEQ ID NO: 140-157 or corresponding sequences having at least 95%, preferably at least 99 % sequence homology with SEQ ID NO: 140-157.

In the sequence list, the sequences marked with the suffix _ID denote primers comprising a unique molecular identifier (UMI) sequence.

The UMI sequence may have the sequence as defined in SEQ ID NO:383. The UMI sequence may have the sequence HHHACHHH.

However, the invention is by no means limited to this UMI sequence.

The "target-specific sequence" (4, 4') is configured to hybridize to a predetermined gene segment or to a region downstream or upstream of the predetermined gene segment 3.

In the first step a) of the method, an individual amplified library 6 is obtained in each well of the first array.

As used herein, the term "amplified library" means an amplified product comprising a plurality of target regions (denoted 7 in figure 1a) comprising or encompassing the predetermined gene segments targeted by the target primers 2,2'. The amplified library 6 further comprises two universal regions, i.e. a reverse universal region 8 and a forward universal region 8'. As illustrated in figure 1a, the target region 7 is arranged between the reverse 8 and the forward 8' universal regions. The amplified library or amplified product may also be referred to as a "multiplexed product".

Multiplexing is a term that refers to multiple samples being processed at the same time. Multiple different DNA sequences are amplified simultaneously in the amplification steps of the method of the present disclosure. Hence, DNA sequences in a plurality of loci in the genomic DNA sample are generated and comprised in the multiplexed product.

The universal regions 8,8' form binding or "target" sites for the well index primers in step b) of the method.

In step b) of the method, the amplified libraries 6 are labelled with well-specific index sequences 11,11' and an array index sequence 16.

This is accomplished by contacting the amplified libraries 6 of step a) with a well-index primer pair 9,9' and an array-index primer pair 10,10'.

This step is performed in a plurality of second arrays.

In the context of the present disclosure, "a plurality of second arrays" means at least two second arrays. Preferably, the plurality of second arrays comprises between 3 and 400 second arrays, more preferably between 10 and 250 different arrays.

The second array may e.g. be a PCR plate.

As illustrated in figure 1a, the "well-index primer pair" contains a reverse well-index primer 9 and a forward well-index primer 9. Each of these well-index primers contains a well-specific sequence (11,11'), a first well-universal sequence (12,12') and a second well-universal sequence (13,13').

As illustrated in figure 1a, the well-specific sequence 11,11' is arranged between the first well-universal sequence 12,12' and the second well-universal sequence 13,13'.

The "well-specific index sequence" is a sequence which allows the library of a specific well and an individual sample to be labelled, and to be subsequently extracted from the sequenced library obtained in step d) of the method.

The first reverse well-universal sequence 12 targets the reverse universal region 8 of the amplified library 6. Likewise, the first forward well-universal sequence 12' targets the forward universal region 8' (see figure 1a).

The second reverse well-universal sequence 13 forms the 5' end of the reverse well index primer 9. The 5' end may also be referred to as the "outer end" of the reverse well index primer 9. The second reverse well-universal sequence 13 enables subsequent labelling with a reverse array-specific index 16. Hence, the second reverse well-universal sequence 13 forms a "bridge" to subsequent "labelling" amplifications.

The second forward well-universal sequence 13' forms the 5' end of the forward well index primer 9'. The 5' end may also be referred to as the "outer end" of the forward well index primer 9'. The second forward well-universal sequence 13' enables subsequent labelling with a forward array-specific index. Hence, the second forward well-universal sequence 13' forms a "bridge" to subsequent amplifications.

The array-index primer pair contains a reverse array-index primer 10 and a forward array-index primer 10'. At least one of these array-index primers contains an array-specific sequence 16. Furthermore, each of the array-index primers 10,10' contains at least one first array-universal sequence 14,14'.

The "array-specific index sequence" is a sequence which allows the library of a specific array, i.e. from a specific second array to be extracted from the sequenced product obtained in step d).

As illustrated in figure 1a, the reverse array index primer 10 comprises a reverse array-universal sequence 14. The reverse array-universal sequence 14 is configured to target the second reverse well-universal sequence 13 of the reverse well-index primer 9.

The forward array index primer 10' comprises a forward array-universal sequence 14'. The forward array-universal sequence 14' is configured to target the second forward well-universal sequence 13' of the forward well-index primer 9'.

Preferably, the reverse array index primer 10 further comprises a second reverse array-universal sequence 15. The second reverse array-universal sequence 15 forms the 5'end of the reverse array index primer 10.

Preferably, the forward array index primer 10' further comprises a second forward array-universal sequence 15'. The second forward array-universal sequence 15' forms the 5' end of the forward array index primer 10'.

In figure 1a, the array-specific index sequence 16 is arranged between the first reverse array-universal sequence 14 and the second reverse array-universal sequence 15.

In the context of the present disclosure, it is equally conceivable that the array-specific index sequence 16 is arranged between the first forward array-universal sequence 14' and the second forward array-universal sequence 15'.

In step b) of the method, a labelled amplified library is obtained.

The term "labelled amplified library" means an amplified product comprising a plurality of target regions complementary to the predetermined gene segments targeted by the target primers, a reverse well index region 18, a forward well index regions 18', and an array-index region 19.

As illustrated in figure 1a, the reverse well index region 18 of the labelled amplified library 17 corresponds to the reverse well-index sequence 11 of the reverse well-index primer 9.

Likewise, the forward well index region 18' of the labelled amplified library 17 corresponds to the forward well-index sequence 11' of the forward well-index primer 9'.

The array-index region 19 corresponds to the array-index sequence 16 of one of the array-index primers 10, 10'.

The well-index primers 9,9' may also be referred to as "inner primers". The array-index primers 10,10' may also be referred to as "outer primers".

The well-index primers are rate-limiting, and are typically used in lower concentrations. The array-index primers bind to the inner primer reaction. The array-index primers are typically used in higher concentrations.

For example, the ratio of the well-index primer pairs (9,9') to the array-index primer pairs (10,10') used in the method 1 may be at least 1: 10, preferably from 1:20 to 1:60.

The reverse well-index primer 9 may have a sequence as defined in any one of SEQ ID NO: 158-253 or a corresponding sequence having at least 95%, preferably at least 99 % sequence homology with any one of SEQ ID NO: 158-253.

The forward well-index primer 9' may have a sequence as defined in any one of SEQ ID NO: 254-349 or a corresponding sequence having at least 95%, preferably at least 99 % sequence homology with any one of SEQ ID NO:254-349.

The reverse array-index primer 10 may have a sequence as defined in any one of SEQ ID NO:350-373 or a corresponding sequence having at least 95%, preferably at least 99 % sequence homology with any one of SEQ ID NO:350-373.

The forward array-index primer 10' may have a sequence as defined in SEQ ID NO: 374 or a corresponding sequence having at least 95%, preferably at least 99 % sequence homology with SEQ ID NO:374.

In step c) of the method, the amplified libraries are combined. Hence, each of the labelled amplified libraries 17 contained in the wells of the plurality of second arrays are combined.

The combined library is an amplified product comprising a plurality of labelled amplified libraries 17.

For example, each of the labelled amplified libraries of the second arrays may be prepared separately by combining equal amounts of each of the labelled amplified libraries and purified in two steps, e.g. by agarose gel purification, followed by magnetic bead purification. One array-specific library may be mixed with additional array-specific libraries, each containing different array-specific indexes but purified using the same steps.

Step d) of the method comprises sequencing the combined library to yield a sequenced library.

The present disclosure is not limited to a specific sequencing technique or a specific sequencing instrument. Any sequencing technique or instrument known to the skilled person may be utilized.

The sequencing is preferably performed by next-generation sequencing (NGS). For example, an Illumina MiSeq or Illumina HiSeq instrument may be used.

The multiple amplifications performed in the various steps of the method may be sequenced in one single run.

The sequencing step allows for the determination of the nucleic acid sequence of each target sequence encoding a predetermined gene segment.

The sequencing step d) may comprise providing a reverse sequencing primer targeting the reverse array-index primer 10 and a forward sequencing primer targeting the forward array-index primer 10'.

Either the first or the second array-universal sequences of the array-index primer pairs 10,10' may be targeted in the sequencing step.

Step e) of the method comprises analyzing the sequenced library obtained in step d). Preferably, this is accomplished by bioinformatic analysis.

A bioinformatic analysis is beneficial to analyze and interpret large data sets. Hence, the method of the present disclosure enables the typing of the genes of the immune system, and the allelic variants of these genes, in a high-throughput and rapid manner.

The bioinformatic analysis may comprise identifying the allelic variants of the immune genes by comparing the target regions 7 of each labelled amplified library 17 with a predefined gene reference set.

The well-specific indexes 11, 11' and array specific indexes 16 allow for bioinformatic separation of the libraries from a multiplexed sequence set that contains multiple separate labelled amplified libraries.

Step e) may be performed by:
- extracting the amplified libraries belonging to a specific second array by using the array-specific index,
- extracting the amplified libraries belonging to a specific well by using the well-specific indexes,
- comparing corresponding target regions of the amplified libraries with a pre-defined reference set.

This allows for the determination of the allelic variants of a specific gene segment and/or a specific gene.

In exemplary embodiments, step e) is performed by a processing unit.

The processing unit may in some embodiments form part of a computer system, further comprising a database. The computer system may be implemented as a server. It may be possible to make use of more than a single processing unit, such as for example including FPGAs, ASICs, etc., software systems and applications, software packages, mechanical and electrical parts, etc. Software packages may be part of the server, the database, and/or a network.

The extracted sub-libraries may be assigned to a database of allelic variants for each of the targeted adaptive immune genes. Accordingly, a rapid processing of the sub-library sequences is accomplished.

The method of the present disclosure may further comprise a step of removing, by the processing unit, target sequences being present at less than 5%, e.g. less than 10% the count of the highest count of target sequences of a predetermined gene segment.

In other words, a minimum number of sequences are required for a specific allele to be considered present in an individual. False positives may be removed using an algorithm that identifies sequences present at less than 5%, e.g. less than 10% the number of other alleles of the same gene ("the allelic ratio filter").

This is based on the principle that almost all autosomal genes in an individual are present in two physical copies, one derived from the mother and the other from the father. Amplification of a genomic segment with allelic variation, such as an adaptive immune gene segment, will be of roughly equal efficiency for each allelic variant of that gene segment. Alleles of that gene should therefore have similar numbers of individual sequences in each sub-library. False positives, for example instrument associated sequence errors, will generally occur at a rate consistent with the expected error rate of the instrument. The error rate of the Illumina MiSeq, for example, is around 1% when dealing with sequences of around 500 bp. Setting an allelic ration filter of less than 5%, e.g. less than 10% removes all variants that are present at less than 5%, e.g. less than 10% the count of the highest count allele of that gene.

The method may further comprise a step of outputting, by the processing unit, the set of target sequences associated with each of the predetermined gene segments targeted.

The set of alleles for each of the genes targeted may thus be outputted.

In embodiments where the libraries comprise UMI sequences, the method may also extract the UMI from each sequence and the number of individual barcodes per allele may be used as a means of ensuring that possible PCR bias is avoided.

In order to enhance the accuracy of the analysis, a control target may be introduced in the initial library targets. The control target may be a non-adaptive immune gene that serves as a control of DNA quantity in each well. If this is not sufficiently amplified (based on counts), the conclusion may be that there was insufficient template DNA in that well, and will not attempt to generate a genotype for that well. Furthermore, if there is sufficient control amplification in a well, the genotype can be determined for that well. Furthermore, the presence of the control target sequences may be utilized to bioinformatically identify copy number variations in target sequences, for example based on discrepant ratios of control/target sequences, i.e., revealing absence of specific target genes in certain cases.

Since the method of the present disclosure works by targeting a predesignated set of genes that can be present in multiple genomic loci, this allows the simultaneous targeting of additional genes, unconnected with the adaptive immune system. These additional targets may be chosen for reasons of low genetic variability and copy number variation (CNV). They function as controls, enabling the computational identification of deletions and duplications in the adaptive immune targets through variations in the ratio of target gene sequence counts to control gene sequence counts.

Exemplary reverse control primers are defined in SEQ ID NO:375-378. Exemplary forward control primers are defined in SEQ ID NO: 379-382.

The method of the present disclosure is not limited to a specific size of the predetermined gene segments. For example, the size of the predetermined gene segment may be up to 1000 kbp. However, to enable a high-throughput analysis, the size of the gene segment targeted may be between 100 and 1000 bp in size, preferably between 300 and 800 bp in size.

A size in the above-mentioned range enables the targeting of variable (V) diversity (D) and junction (J) segments of B and T- cell receptor genes, in addition to exons of other genes involved in the adaptive immune system, e.g. MHC gene exons.

The size of a labelled amplified library obtained in step b) of the method may be in the range of from 400 pb to 1000 bp, e.g. from 400 to 800 bp.

In exemplary embodiments, at least one of the reverse 2 or forward 2' target primers of the set of target primers further comprises a unique molecular identifier UMI sequence.

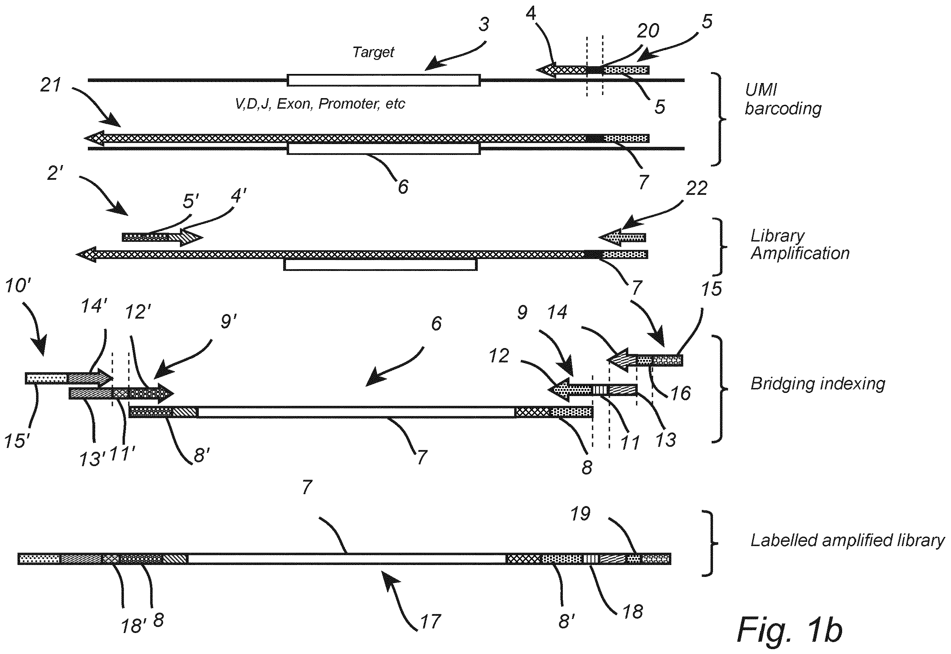

A method involving the utilization of a UMI sequence is schematically illustrated in figure 1b.

In this method, the first amplification step a) may be divided into two separate steps (two separate amplifications).

As illustrated in figure 1b, one of the target primers 2 (the reverse target primer) comprises a target sequence 4, a UMI sequence 20, and a universal sequence 5. The UMI sequence 20 is preferably arranged between the target sequence 4 and the universal sequence 5.

First, an amplified product 21 comprising a target region (comprising the targeted pre-determined gene segments (3)) is formed by utilizing the reverse target primer.

Subsequently, a forward target primer 2' is used. The forward target primer comprises a forward target sequence 4' and a universal sequence 5' arranged 5' of the forward target sequence 4'. Furthermore, in this separate amplification step, a conventional primer 22 containing a universal sequence may be used. This universal sequence corresponds to the universal sequence 5 of the reverse target primer 2.

The subsequent step of labelling the amplified libraries 6 is performed in the same manner as described hereinbefore with respect to figure 1a.

The method is by no means limited to the UMI barcoding step illustrated in figure 1b.

The method of the present disclosure may, as mentioned hereinbefore, comprise various purification steps, e.g. to remove unincorporated primers.

Purification steps may be introduced after step a) of providing a first set of amplified libraries, after step b) of labelling the amplified libraries, and/or after step c) of combining the labelled amplified libraries.

For example, a column-based process (i.e. Qiagen PCR cleanup columns) or a magnetic bead based process (i.e. AMPure XP bead purification) may be utilized. However, any suitable purification process known to the skilled person may be used.

In the various amplifications performed in the method of the present disclosure, the DNA samples; i.e. DNA templates are denatured at an elevated temperature, followed by annealing at an elevated temperature and extension at an elevated temperature.

For example, exemplary PCR conditions used for step a) of providing the first set of amplified libraries 5 may be initial denaturing at 98°C for about 5 minutes, followed by multiple cycles (e.g. 20-30 cycles) of denaturing at 98°C for about 30 seconds, annealing at 66°C for about 30 seconds, extension at 72°C for about 30 seconds, followed by 7 minutes at 72°C and then 4°C.

For example, the PCR conditions for the labelling step b) may be initial denaturing at 98°C for about 5 minutes, followed by multiple cycles (e.g. 5-10 cycles) of denaturing at 98°C for about 30 seconds, annealing at 66°C for about 30 seconds, extension at 72°C for about 30 seconds, followed by 7 minutes at 72°C and then 4°C.

For example, the PCR conditions used for the introduction of the UMI sequence may be initial denaturing at 98°C for about 5 minutes, followed by a single annealing cyle at 66°C for about 2 minutes, and a single extension cycle at 72°C for about 3 minutes.

The PCR conditions mentioned hereinabove are purely exemplary, and the invention is by no means limited to these conditions. The skilled person in the field is well aware of suitable amplification conditions.

According to another aspect, there is provided a kit for use in a method of typing the immune genes and the allelic variants thereof, wherein the kit comprises:
- a set of target primers comprising a plurality of reverse target primers 2; each reverse target primer targeting the 3' end of a pre-determined gene segment 3 of an immune gene, and a plurality of forward target primers 2'; each forward target primer 2' targeting the 5' end of the pre-determined gene segment 3, wherein each of the reverse target primers 2 comprises a reverse target sequence 4 and a reverse universal sequence 5 arranged 5' of the reverse target sequence 4, wherein the reverse universal sequence 5 is common to all reverse target primers 2 in the set of target primers, wherein each of the forward target primers 2' comprises a forward target sequence 4' and a forward universal sequence 5' arranged 5' of the forward target sequence 4', wherein the forward universal sequence 5' is common to all forward target primers 2 in the set of target primers;
- a plurality of well-index primer pairs (9,9'), wherein each well-index primer pair (9,9') comprises a reverse well-index primer 9 and a forward well-index primer 9'; the reverse well-index primer 9 comprising a first reverse well-universal sequence 12 corresponding to the reverse universal sequence 5 of the reverse target primer 2, a second reverse well-universal sequence 13, and a reverse well-specific index sequence 11 arranged between the first 12 and second 13 reverse well-universal sequences; the forward well-index primer 9' comprising a first forward well-universal sequence 12' corresponding to the forward reverse universal sequence 5' of the forward target primer 2', a second forward well-universal sequence 13', and a forward well-specific index sequence 11' arranged between the first 12' and second 13' forward well-universal sequences,
- a plurality of array-index primer pairs (10,10') each comprising a reverse array-index primer 10 and a forward array-index primer 10'; the reverse array-index primer 10 comprising at least a first reverse array-universal sequence 14 targeting the second reverse well-universal sequence 13; the forward array-index primer 10' comprising at least a first forward array-universal sequence 14' targeting the second forward well-universal sequence 13', wherein at least one of the reverse 10 and forward 10' array-index primers comprises an array-specific index sequence 16 arranged 5' of the a first reverse array-universal sequence 14 or the first forward array-universal sequence 14'.

The kit may be used in the method described hereinbefore.

According to yet another aspect, there is provided a set of target primers comprising a plurality of reverse target primers 2 and a plurality forward target primers 2' for use in a method of typing the immune genes, preferably the genes of the adaptive immune system, and the allelic variants thereof, wherein
the set of target primers is configured to target the IGHD genes and comprises the reverse target primers defined by SEQ ID NO:1-13 or corresponding sequences having at least 95 %, preferably at least 99% sequence homology with SEQ ID NO:1-13, and the forward target primers defined by SEQ ID NO:14-31 or corresponding sequences having at least 95%, preferably at least 99 % sequence homology with SEQ ID NO:14-31, and/or wherein
the set of target primers is configured to target the IGHJ genes and comprises the reverse target primers defined by SEQ ID NO: 32-36 or corresponding sequences having at least 95%, preferably at least 99 % sequence homology with SEQ ID NO:32-36, and the forward target primers defined by SEQ ID NO: 37-41 or corresponding sequences having at least 95%, preferably at least 99 % sequence homology with SEQ ID NO:37-41, and/or wherein
the set of target primers is configured to target the IGHV genes and comprises the reverse target primers defined by SEQ ID NO:42-74 or corresponding sequences having at least 95%, preferably at least 99 % sequence homology with SEQ ID NO:42-74, and the forward target primers defined by SEQ ID NO: 75-121 or corresponding sequences having at least 95%, preferably at least 99 % sequence homology with SEQ ID NO:75-121, and/or wherein
the set of target primers is configured to target the TRG genes and comprises the reverse target primers defined by SEQ ID NO:122-139 or corresponding sequences having at least 95%, preferably at least 99 % sequence homology with SEQ ID NO:122-139, and the forward target primers defined by SEQ ID NO: 140-157 or corresponding sequences having at least 95%, preferably at least 99 % sequence homology with SEQ ID NO:140-157.

Table 1 hereinbelow list exemplary target primers including their names and SEQ ID number.

**Table 1: Exemplary reverse and forward target primers**

| Name | SEQ NO: | Name | SEQ NO: | Name | SEQ NO: |
|---|---|---|---|---|---|
| IGHD1-14BR_ID | 1 | IGHD5-24FG | 27 | IGHV2-70*01_R | 53 |
| IGHD1-14R_ID | 2 | IGHD6-13F | 28 | IGHV3-11*01_R | 54 |
| IGHD1-7R_ID | 3 | IGHD6-13FC | 29 | IGHV3-13R | 55 |
| IGHD2-2R_ID | 4 | IGHD6-13F2 | 30 | IGHV3-15R_96 | 56 |
| IGHD3-22RC_ID | 5 | IGHD6-25F | 31 | IGHV3-20*01_R | 57 |
| IGHD3-9_10R_ID | 6 | IGHJ1J2D727R2_ID | 32 | IGHV3-23*01_R | 58 |
| IGHD4-11R_ID | 7 | IGHJ3R_ID | 33 | IGHV3-30_Gen_R | 59 |
| IGHD4-17R_ID | 8 | IGHJ4R_ID | 34 | IGHV3-43*01_R | 60 |
| IGHD4-23RB_ID | 9 | IGHJ5R_ID | 35 | IGHV3-43D*03_R | 61 |
| IGHD5-24RG_ID | 10 | IGHJ6R_ID | 36 | IGHV3-49R | 62 |
| IGHD6-19R_ID | 11 | IGHJ1J2D727F2C | 37 | IGHV3-53R | 63 |
| IGHD6-25R_ID | 12 | IGHJ3F | 38 | IGHV3-64*01_R | 64 |
| IGHD6-6R_ID | 13 | IGHJ4F | 39 | IGHV3-64D*06_R | 65 |
| IGHD1-20_1-26F | 14 | IGHJ5F | 40 | IGHV3-74*01_R | 66 |
| IGHD1-7F | 15 | IGHJ6F | 41 | IGHV3-9*01_R | 67 |
| IGHD1FC | 16 | IGHV1-18*01_R | 42 | IGHV4-30-4*01_R | 68 |
| IGHD2-21F | 17 | IGHV1-2R_1 | 43 | IGHV4-34R_96 | 69 |
| IGHD2-2F | 18 | IGHV1-3*01_R | 44 | IGHV4-39*01_R | 70 |
| IGHD2-8_15F | 19 | IGHV1-3R1 | 45 | IGHV4-59*01_R | 71 |
| IGHD3-22FB | 20 | IGHV1-45*02_R | 46 | IGHV5-10-1*01_ID_R | 72 |
| IGHD3-22FC | 21 | IGHV1-46*01_R | 47 | IGHV6-1R | 73 |
| IGHD3-3FB | 22 | IGHV1-58*01_R | 48 | IGHV7-4-1_R | 74 |
| IGHD3-9_10F | 23 | IGHV1-69_3_R | 49 | IGHV1-18*01F | 75 |
| IGHD4-11F | 24 | IGHV1-69-2*01_R | 50 | IGHV1-24_F2 | 76 |
| IGHD4-23F | 25 | IGHV1-8*01_R | 51 | IGHV1-2F1 | 77 |
| IGHD4-4F | 26 | IGHV2-26*01_R | 52 | IGHV1-2F2 | 78 |
| IGHV1-3F1 | 79 | IGHV3-64*01F | 106 | TRGV1R_ID | 133 |
| IGHV1-3F2 | 80 | IGHV3-64D*06F | 107 | TRGV2R_ID | 134 |
| IGHV1-45-58F | 81 | IGHV3-72*01F | 108 | TRGV3R_ID | 135 |
| IGHV1-46_F2 | 82 | IGHV3-73*01F | 109 | TRGV4R_ID | 136 |
| IGHV1-46F1 | 83 | IGHV3-74*01F | 110 | TRGV5R_ID | 137 |
| IGHV1-58_F1 | 84 | IGHV3-7F | 111 | TRGV8R_ID | 138 |
| IGHV1-69-2*01F | 85 | IGHV3-9*01F | 112 | TRGV9R2_ID | 139 |
| IGHV1-69F1 | 86 | IGHV4-30-2*01F | 113 | TRGC1EX12F | 140 |
| IGHV1-69F2 | 87 | IGHV4-30-4*01F | 114 | TRGC1EX1F | 141 |
| IGHV1-8_F1 | 88 | IGHV4-34F_96 | 115 | TRGC1EX3F | 142 |
| IGHV1-8F2 | 89 | IGHV4-38-2*01F | 116 | TRGC2EX1F | 143 |
| IGHV2-26F | 90 | IGHV4-4F | 117 | TRGC2EX2F | 144 |
| IGHV2-5*01F | 91 | IGHV5-10-1*01F | 118 | TRGC2EX3F | 145 |
| IGHV2-5F | 92 | IGHV6-1F | 119 | TRGC2EX4F | 146 |
| IGHV2-70*01F | 93 | IGHV7-4-1_F2 | 120 | TRGJ1_2F | 147 |
| IGHV2-70F | 94 | IGHV1-45_F2 | 121 | TRGJP_F | 148 |
| IGHV3-11F1 | 95 | TRGC1EX12R_ID | 122 | TRGJP1_F | 149 |
| IGHV3-13F1 | 96 | TRGC1EX1R_ID | 123 | TRGJP2_F | 150 |
| IGHV3-15F2 | 97 | TRGC1EX3R_ID | 124 | TRGV1F | 151 |
| IGHV3-20F | 98 | TRGC2EX1R_ID | 125 | TRGV2F | 152 |
| IGHV3-21*01F | 99 | TRGC2EX2R_ID | 126 | TRGV3F | 153 |
| IGHV3-23*01F | 100 | TRGC2EX3R_ID | 127 | TRGV4F | 154 |
| IGHV3-30F_96 | 101 | TRGC2EX4R_ID | 128 | TRGV5F | 155 |
| IGHV3-30F1 | 102 | TRGJ1_2R_ID | 129 | TRGV8F | 156 |
| IGHV3-43F2 | 103 | TRGJP_R_ID | 130 | TRGV9F2 | 157 |
| IGHV3-49F | 104 | TRGJP1_R_ID | 131 | | |
| IGHV3-53F | 105 | TRGJP2_R_ID | 132 | | |

In table 1, the letter "R" denotes the reverse target primers, and the letter "F" denotes the forward target primers. The suffix "_ID" denotes the incorporation of a UMI sequence.

### Examples

### Example 1

### Library amplification

4 ng of DNA template from 8 individuals was amplified as follows. The DNA template was placed in the wells of a 96 well PCR array plate and a library mastermix of 10 microlitres was added to each well and mixed. This mastermix contained 6 microlitres of 2 x High Fidelity PCR mix (including PCR buffer and NTPs), 3.5 microlitres of water, and 0.5 microlitres of the library primer set mix. For the IGHV genotyping, the primer set consisted of ImmuneDiscover IGHV target set reverse and IGHV forward primer set (as defined in SEQ ID NO:42-74, and SEQ ID NO:75-121, respectively), including the control primers that target the genes HOXB5, on human chromosome 17, Rennin on human chromosome 1, BTNL3 on chromosome 5 and CCR5, on human chromosome 3. The reverse control primers are defined by SEQ ID NO:375-378. The forward control primers are defined by SEQ ID NO: 379-382.

The following PCR conditions were used:
- 5 minutes at 96 degrees C, followed by 30 cycles of
- Denaturing at 98 degrees C for 30 seconds,
- Annealing at 66 degrees C for 30 seconds,
- Extension at 72 degrees for 30 seconds, and
- Final extension at 72 degrees for 5 minutes.

Following amplification, the amplified libraries were purified using magnetic beads, to remove excess primers, with the resultant purified library product being used as template for the bridge indexing reaction.

### Bridge Indexing

The bridge indexing protocol enables the simultaneous use of four primers to introduce three indices to each amplified library sequence. The protocol works on the principle that a rate limiting primer concentration can be utilized in the early stages of a reaction that is then completed by an additional primer set that recognizes targets created by the earlier rate limiting primer amplification. The rate limiting primers therefore create a bridge between the target (in this case the ImmuneDiscover library product) and the outer primers that would otherwise not recognize the library product.

The rate limiting primers, termed the `well index primers', are designed such that they are paired and specific to each single well of the 96 well plate. One primer, the forward well index primer, recognizes the forward universal sequence introduced during library PCR and the other, the reverse well index primer, recognizes the reverse universal sequence. 96 pairs of forward well index primers and reverse well index primers were used.

The central section of each of the well index primers contained a 6 bp index that is unique to that primer such that each well will have a unique forward well index and a unique reverse well index, both of which are specific to one well out of the 96. The outer part of the forward well index primer contained a target site for a forward sequencing primer while the reverse contained the target site for a reverse sequencing primer and a separate index, termed the plate index. These two outer primers are termed the "plate indexing primers" (or array-index primers).

4 microlitres of the Ampure purified library product, present in a 96 well plate was transferred to each well of the bridge index plate. The bridge index plate contained 2 microlitres of primer mix consisting of 0.025 µM of the well index primers (the pair being specific for each of the 96 wells) and 1 µM of the plate index primers. 6 microlitres of the 2 x High Fidelity PCR mix was added, the samples mixed, sealed and amplified for 11 cycles of:
- 95 degrees C for 5 minute, initial denaturing.
- 98 degrees for 30 seconds, denaturing step
- 66 degrees for 30 seconds, annealing step
- 72 degrees for 30 seconds, extension step
- 72 degrees for 5 minutes final extension.

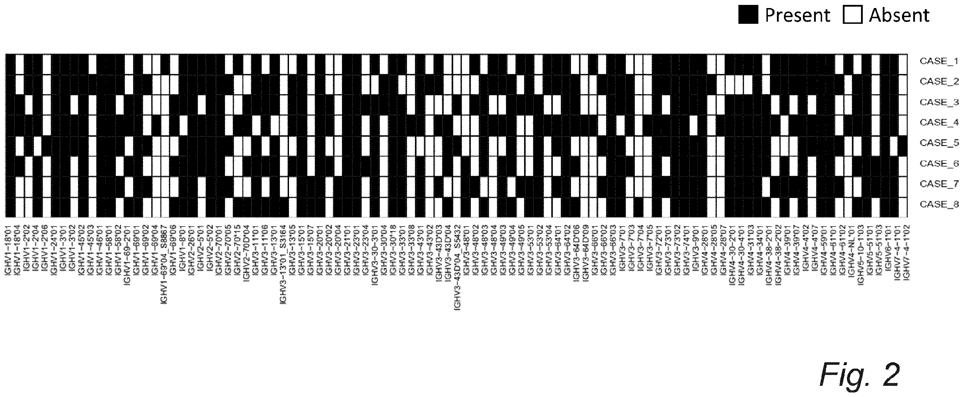

Following this step, the resultant indexed libraries were combined and sequenced using the Illumina MiSeq system. The resultant sequence data was analyzed using the ImmuneDiscover software to produce the individualized IGHV genotype shown in Figure 2. In figure 2, all alleles present are shown by dark boxes, and the absence of an allele is shown with a white box. IGHV allele names are shown below the figure and the case numbers are shown on the right. These results demonstrate that the method of the present disclosure can reveal highly individual IGHV genotypes in all cases analyzed.

### Example 2

In order to demonstrate the reproducibility of the ImmuneDiscover genotype analysis, genotyping of the TCR Gamma variable genes was performed.

Template and library and indexing conditions were the same as for the IGHV library of Example 1, except the TRG reverse primer set and TRG forward primer set (as defined in SEQ ID NO: 122-139, and SEQ ID NO: 140-157, respectively) was used in this library amplification. 50 individual replicates of the 8 cases were used for genotyping, and the results are illustrated in Figure 3.

All alleles that were present in each case are shown with a dark shaded box and all alleles that were absent are shown with a light shaded box.

These results indicate that the same genotype is present in each of the 50 replicates for each of the 8 cases, demonstrating that the method of the present disclosure produces highly reproducible genotypes.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

The control functionality of the present disclosure may be implemented using existing computer processors, or by a special purpose computer processor for an appropriate system, incorporated for this or another purpose, or by a hardwired system. Embodiments within the scope of the present disclosure include program products comprising machine-readable media for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, CD-ROM or other optical disk storage, magnetic disk storage or other magnetic storage devices, or any other medium which can be used to carry or store desired program code in the form of machine-executable instructions or data structures and which can be accessed by a general purpose or special purpose computer or other machine with a processor. When information is transferred or provided over a network or another communications connection (either hardwired, wireless, or a combination of hardwired or wireless) to a machine, the machine properly views the connection as a machine-readable medium. Thus, any such connection is properly termed a machine-readable medium. Combinations of the above are also included within the scope of machine-readable media. Machine-executable instructions include, for example, instructions and data which cause a general-purpose computer, special purpose computer, or special purpose processing machines to perform a certain function or group of functions. Also, two or more steps may be performed concurrently or with partial concurrence. Such variation will depend on the software and hardware systems chosen and on designer choice. All such variations are within the scope of the disclosure. Likewise, software implementations could be accomplished with standard programming techniques with rule-based logic and other logic to accomplish the various connection steps, processing steps, comparison steps and decision steps.

## Claims

1. A method (1) for typing immune genes and allelic variants thereof comprising
a) providing a set of amplified libraries (6) in at least one first array comprising a plurality of wells by performing an individual amplification reaction in each well of said first array(s); said individual amplification reaction comprising contacting a genomic DNA sample with a polymerase and a set of target primers; said set of target primers comprising a plurality of reverse target primers (2); each reverse target primer targeting the 3' end of a pre-determined gene segment (3) of an immune gene, and a plurality of forward target primers (2'); each forward target primer targeting the 5' end of said pre-determined gene segment (3), wherein
each of said reverse target primers (2) comprises a reverse target sequence (4) and a reverse universal sequence (5) arranged 5' of said reverse target sequence (4), wherein said reverse universal sequence (5) is common to all reverse target primers (2) in said set of target primers, wherein
each of said forward target primers (2') comprises a forward target sequence (4') and a forward universal sequence (5') arranged 5' of said forward target sequence (4'), wherein said forward universal sequence (5') is common to all forward target primers (2) in said set of target primers;
whereby an individual amplified library (6) is obtained in each well; said amplified library (6) comprising a plurality of target regions (7) comprising the targeted pre-determined gene segments (3), a reverse universal region (8) corresponding to said reverse universal sequence (5), and a forward universal region (8') corresponding to said forward universal sequence (5');
b) labelling each amplified library (6) of said set of amplified libraries (6) in a plurality of second arrays; each of said second arrays comprising a plurality of wells, by performing an individual amplification reaction in each well of said second arrays; said individual amplification reaction comprising contacting said amplified library (6) with a polymerase, a well-index primer pair (9,9'), and an array-index primer pair (10,10'), wherein
said well-index primer pair (9,9') comprises a reverse well-index primer (9) and a forward well-index primer (9'); said reverse well-index primer (9) comprising a first reverse well-universal sequence (12) targeting said reverse universal region (8), a second reverse well-universal sequence (13), and a reverse well-specific index sequence (11) arranged between said first (12) and second (13) reverse well-universal sequences; said forward well-index primer (9') comprising a first forward well-universal sequence (12') targeting said forward universal region (8'), a second forward well-universal sequence (13'), and a forward well-specific index sequence (11') arranged between said first (12') and second (13') forward well-universal sequences, wherein
said array-index primer pair (10,10') comprises a reverse array-index primer (10) and a forward array-index primer (10'); said reverse array-index primer (10) comprising at least a first reverse array-universal sequence (14) targeting said second reverse well-universal sequence (13); said forward array-index primer (10') comprising at least a first forward array-universal sequence (14') targeting said second forward well-universal sequence (13'), wherein at least one of said reverse (10) and forward (10') array-index primers comprises an array-specific index sequence (16) arranged 5' of said first reverse array-universal sequence (14) or said first forward array-universal sequence (14'); wherein
different well-index primer pairs (9,9') are used in each well of said second arrays, and wherein identical array-index primer pairs (10, 10') are used in each well of each individual second array of said plurality of second arrays,
whereby a labelled amplified library (17) is obtained in each well,
c) combining the labelled amplified libraries (17) of each well of said plurality of second arrays to yield a combined library;
d) sequencing said combined library to yield a sequenced library;
e) analyzing said sequenced library.

2. The method (1) according to claim 1, wherein said reverse array-index primer (10) further comprises a second reverse universal sequence (15) arranged 5' of said first reverse array-universal sequence (14); said forward array-index primer (10') further comprising a second forward array-universal sequence (15') arranged 5' of said first forward array-universal sequence (14'), wherein said array-specific index sequence (16) is arranged between said first (14) and second (15) reverse array-universal sequences or between said first (14') and second (15') forward array-universal sequences.

3. The method according to any one of the preceding claims, wherein said sequencing step d) comprises providing a forward sequencing primer targeting said reverse array-index primer (10) and a forward sequencing primer targeting said forward array-index primer (10').

4. The method (1) according to claim 1, wherein said plurality of second arrays comprises between 3 and 400, preferably between 10 and 250 different second arrays.

5. The method (1) according to any one of the preceding claims, wherein said immune genes are genes of the adaptive immune system.

6. The method according any one of the preceding claims, wherein said immune genes are B-cell receptor (BCR) genes, T-cell receptor (TCR) genes or major histocompatibility (MHC) genes.

7. The method (1) according any one of the preceding claims, wherein said at least one predetermined gene segment (3) comprises a gene segment of the variable (V), diversity (D) or the junction (J) or constant (C) B-cell or T-cell receptor genes, or MHC gene exons.

8. The method (1) according to any one of the preceding claims, wherein said pre-determined gene segment (3) of said immune gene is between 100 and 1000 bp in size, preferably between 300 and 800 bp in size.

9. The method (1) according to any one of the preceding claims, wherein the ratio of said well-index primer pairs (9,9') to said array-index primer pairs (10,10') used in said method (1) is at least 1:10, preferably from 1:20 to 1:60.

10. The method (1) according to any one of the preceding claims, wherein said set of target primers comprises from 5 to 250, preferably from 15 to 150 reverse and forward target primers (2,2').

11. The method (1) according to any one of the preceding claims, wherein at least one of said reverse (2) or forward (2') target primers of said set of target primers further comprises a unique molecular identifier (UMI) sequence.

12. The method (1) according to any one of the preceding claims, wherein said step e) comprises a bioinformatic analysis of the data obtained in said step d), wherein said bioinformatic analysis comprises identifying the allelic variants of the immune genes by comparing the target regions (7) of each labelled amplified library (17) with a predefined gene reference set.

13. The method (1) according to claim 12, wherein said step e) is performed by a processing unit.

14. A kit for use in a method of typing immune genes and allelic variants thereof, wherein said kit comprises:
- a set of target primers comprising a plurality of reverse target primers (2); each reverse target primer targeting the 3' end of a pre-determined gene segment (3) of an immune gene, and a plurality of forward target primers (2'); each forward target primer (2') targeting the 5' end of said pre-determined gene segment (3), wherein each of said reverse target primers (2) comprises a reverse target sequence (4) and a reverse universal sequence (5) arranged 5' of said reverse target sequence (4), wherein said reverse universal sequence (5) is common to all reverse target primers (2) in said set of target primers, wherein each of said forward target primers (2') comprises a forward target sequence (4') and a forward universal sequence (5') arranged 5' of said forward target sequence (4'), wherein said forward universal sequence (5') is common to all forward target primers (2) in said set of target primers;
- a plurality of well-index primer pairs (9,9'), wherein each well-index primer pair (9,9') comprises a reverse well-index primer (9) and a forward well-index primer (9'); said reverse well-index primer (9) comprising a first reverse well-universal sequence (12) corresponding to said reverse universal sequence (5) of said reverse target primer (2), a second reverse well-universal sequence (13), and a reverse well-specific index sequence (11) arranged between said first (12) and second (13) reverse well-universal sequences; said forward well-index primer (9') comprising a first forward well-universal sequence (12') corresponding to said forward reverse universal sequence (5') of said forward target primer (2'), a second forward well-universal sequence (13'), and a forward well-specific index sequence (11') arranged between said first (12') and second (13') forward well-universal sequences,
- a plurality of array-index primer pairs (10,10') each comprising a reverse array-index primer (10) and a forward array-index primer (10'); said reverse array-index primer (10) comprising at least a first reverse array-universal sequence (14) targeting said second reverse well-universal sequence (13); said forward array-index primer (10') comprising at least a first forward array-universal sequence (14') targeting said second forward well-universal sequence (13'), wherein at least one of said reverse (10) and forward (10') array-index primers comprises an array-specific index sequence (16) arranged 5' of said a first reverse array-universal sequence (14) or said first forward array-universal sequence (14').

15. A set of target primers comprising a plurality of reverse target primers (2) and a plurality forward target primers (2') for use in a method of typing the immune genes, preferably the genes of the adaptive immune system, and the allelic variants thereof, wherein
said set of target primers is configured to target the IGHD genes and comprises the reverse target primers defined by SEQ ID NO:1-13 or corresponding sequences having at least 95 %, preferably at least 99% sequence homology with SEQ ID NO:1-13, and the forward target primers defined by SEQ ID NO:14-31 or corresponding sequences having at least 95%, preferably at least 99 % sequence homology with SEQ ID NO:14-31, and/or wherein
said set of target primers is configured to target the IGHJ genes and comprises the reverse target primers defined by SEQ ID NO: 32-36 or corresponding sequences having at least 95%, preferably at least 99 % sequence homology with SEQ ID NO:32-36, and the forward target primers defined by SEQ ID NO: 37-41 or corresponding sequences having at least 95%, preferably at least 99 % sequence homology with SEQ ID NO:37-41, and/or wherein
said set of target primers is configured to target the IGHV genes and comprises the reverse target primers defined by SEQ ID NO:42-74 or corresponding sequences having at least 95%, preferably at least 99 % sequence homology with SEQ ID NO:42-74, and the forward target primers defined by SEQ ID NO: 75-121 or corresponding sequences having at least 95%, preferably at least 99 % sequence homology with SEQ ID NO:75-121, and/or wherein
said set of target primers is configured to target the TRG genes and comprises the reverse target primers defined by SEQ ID NO:122-139 or corresponding sequences having at least 95%, preferably at least 99 % sequence homology with SEQ ID NO:122-139, and the forward target primers defined by SEQ ID NO: 140-157 or corresponding sequences having at least 95%, preferably at least 99 % sequence homology with SEQ ID NO:140-157.
